# FASCICULE DE BREVET EUROPEEN

(11) **EP 2 069 362 B1**
(45) Date de publication et mention de la délivrance du brevet: **09.07.2014**
(21) Numéro de dépôt: 07848250.2
(22) Date de dépôt: 19.09.2007
(51) Int. Cl.: C07D 498/08

(54) **PROCEDE DE PREPARATION D'HALOGENURES DE N-ALKYL NALTREXONE**
VERFAHREN ZUR HERSTELLUNG VON N-ALYKL-NALTREXON-HALIDEN
PROCESS FOR PREPARING N-ALKYL NALTREXONE HALIDES

(30) Priorité: 21.09.2006 FR 0608286
(43) Date de publication de la demande: 17.06.2009
(73) Titulaire: SANOFI, 75008 Paris (FR)
(72) Inventeur: DLUBALA, Alain, 75013 Paris (FR)
(74) Mandataire: Weber, Mathieu
(86) Numéro de dépôt international: PCT/FR2007/001516
(87) Numéro de publication internationale: WO 2008/034973

(56) Documents cités:
- WO-A-2004/043964
- WO-A-2006/127898
- US-A- 4 176 186
- R. KRASSNIG, M. KOCH, H. K. JENNEWEIN, E. GREINER, H. SCHMIDHAMMER: "A new and efficient synthesis of the u opioid receptor antagonists 14-O-methyl- and 14-O-ethylnaloxone and -naltrexone" HETEROCYCLES, vol. 47, no. 2, 1998, XP001536216

## Description

La présente invention se rapporte à un procédé de préparation d'halogénures de N-alkyl naltrexone.

Les dérivés quaternaires N-alkyle de la naltrexone (une nomenclature de la naltrexone étant (5a)-17-(cyclopropylmethyl)-4,5-epoxy-3,14-dihydroxymorphinan-6-one ou encore N-cyclopropylmethyl-noroxymorphone) sont connus pour leurs applications thérapeutiques, notamment, la N-methyl naltrexone, dont l'utilisation permet d'accompagner un traitement morphinique chez un patient en réduisant de manière significative les effets secondaires indésirables de la morphine et ses dérivés, notamment au niveau du tractus gastrointestinal.

Par N-methyl naltrexone, on entend plus particulièrement la (R)-N-methyl naltrexone c'est-à-dire le composé de configuration (R) vis-à-vis de l'atome d'azote, étant bien connu de l'homme du métier que la (S)-N-methyl a une activité opposée à celle recherchée pour accompagner un traitement morphinique.

La configuration de l'ammonium quaternaire de la N-methyl Naltrexone de formule suivante a été déterminée par RMN ¹H des diastéréoisomères isolés (R) et (S) : 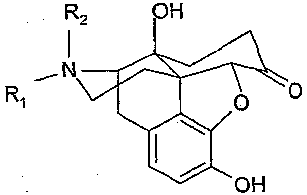
- configuration (S) de l'ammonium (méthyl équatorial) : R₁ représente un groupe méthyle et R₂ représente un groupe méthylcyclopropyle, et
- configuration (R) de l'ammonium (méthyl axial) : R₂ représente un groupe méthyle et R₁ représente un groupe méthylcyclopropyle.

Les déplacements chimiques en RMN ¹H du groupe méthyle (référence TMS ou tétraméthylsilane) sont à 3,62 ppm pour la configuration (R) et à 3,13 ppm pour la configuration (S).

Le brevet US 4176 186 (Boehringer Ingelheim GmbH) décrit des dérivés quaternaires de noroxymorphone ainsi que des procédés de préparations de ceux-ci. Toutefois, les procédés décrits comprennent des conditions, notamment de pression, de quantité nécessaire de réactif, de conversion par échange d'anion sur colonne, non compatibles avec l'application industrielle recherchée.

La demande de brevet WO 2004/043964 A2 décrit un procédé à des pressions moins élevées, comprenant l'utilisation d'un système solvant anhydre, notamment la 1-methyl-2-pyrrolidone, mais qui présente toutefois encore des inconvénients en termes d'impuretés dont la nécessaire teneur suffisamment basse conduit inévitablement à un rendement non satisfaisant.

Il était ainsi d'un intérêt sans cesse croissant de pouvoir disposer d'un procédé permettant de produire à l'échelle industrielle de tels dérivés, dans les meilleures conditions de production (sécurité, environnement) et de rendement.

On a maintenant trouvé de manière tout à fait surprenante et inattendue un procédé permettant d'améliorer de manière très avantageuse à la fois les conditions de mise en oeuvre en termes de sécurité, tant pour le personnel que pour l'environnement, et le rendement pour le produit final souhaité, à savoir un halogénure de N-alkyl naltrexone, en particulier le bromure de N-methyl naltrexone.

Conformément à l'invention, on peut mettre en oeuvre un procédé comprenant les étapes selon le schéma 1 suivant.

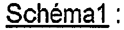

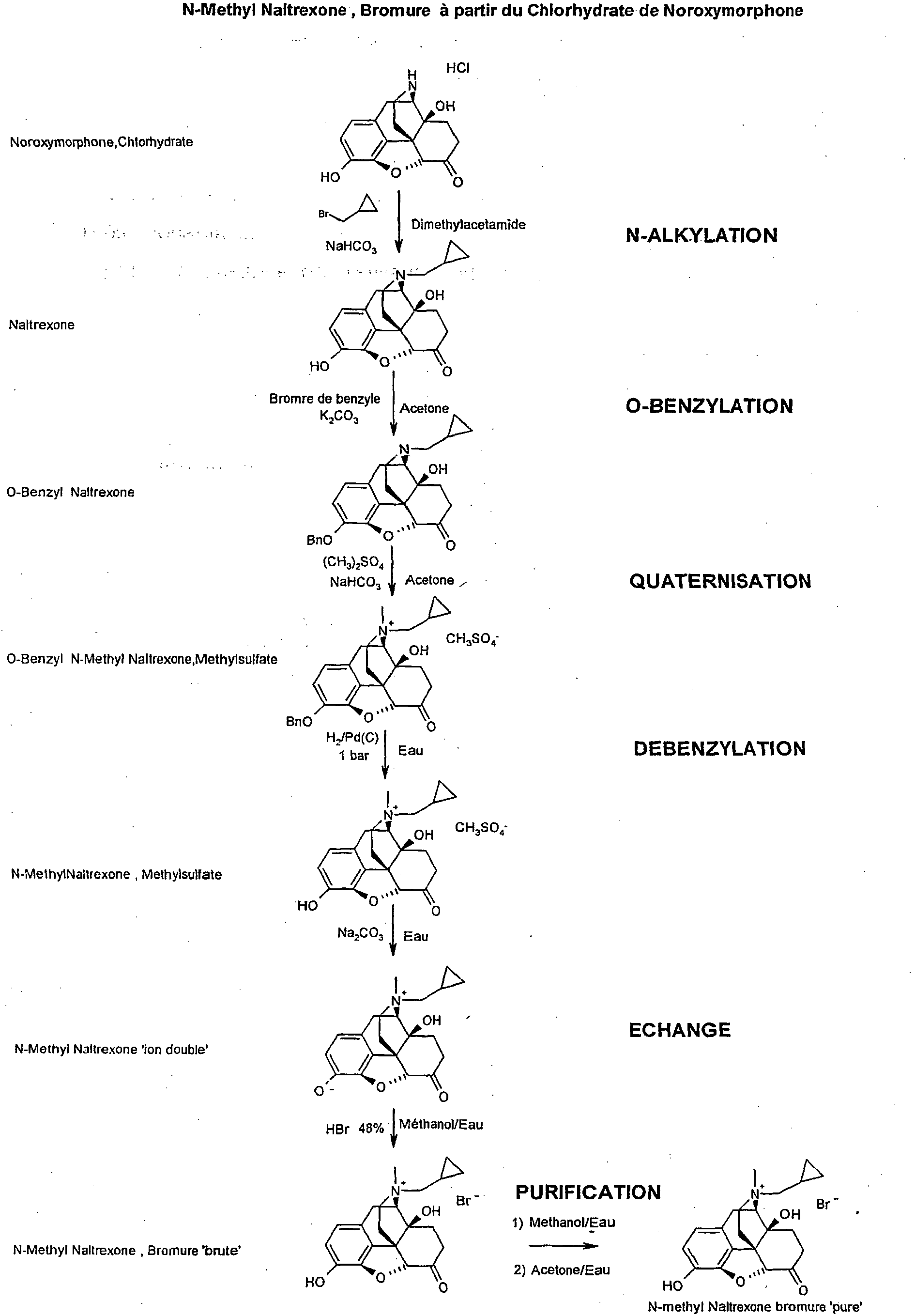

Dans ce qui suit, les composés de départ et les réactifs cités pour le procédé selon l'invention, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

La présente invention a ainsi tout particulièrement pour objet un composé intermédiaire nouveau qui, sans vouloir toutefois être lié à une quelconque théorie, se présente sous forme d'un ion double ayant la formule (I) suivante (qui peut ainsi être dénommé ion double N-methyl naltrexone) :

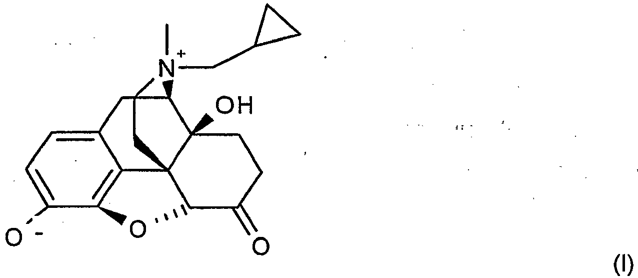

Les diastéréoisomères respectifs de configurations (R) et (S) vis-à-vis de l'atome d'azote de l'ion double N-methyl naltrexone, ainsi que leur mélanges, y compris les mélanges racémiques, font partie de l'invention.

Outre sa forme anhydre, l'ion double N-methyl naltrexone peut également exister à l'état d'hydrate.

Par hydrate, on entend selon l'invention une forme d'association ou de combinaison du composé de formule (I) avec une ou plusieurs molécules d'eau de cristallisation dans le réseau cristallin, c'est-à-dire à l'exclusion de l'eau d'insertion dans les micro-canaux des cristaux (ou «eau d'imprégnation »), l'hydrate pouvant être déterminé d'abord par analyse sur monocristal puis vérifié en routine par analyse comparative de diffractogrammes (ou diagramme de poudre), comme bien connu de l'homme du métier et illustré à l'exemple 1.

De tels hydrates font également partie de l'invention. Par exemple, on peut nommer les formes hémihydrate, dihydrate et trihydrate.

Selon un mode de réalisation particulier de l'invention, l'ion double de formule (I) présente une configuration (R) vis-à-vis de l'atome d'azote et est à l'état dihydrate.

Ce composé nouveau ion double N-methyl naltrexone, de formule (I), peut avantageusement être préparé par un procédé comprenant l'étape consistant à faire réagir du methylsulfate de N-methyl naltrexone en solution aqueuse avec un agent alcalin choisi dans le groupe constitué par le carbonate de sodium (Na₂CO₃), le carbonate de potassium, le carbonate de calcium, le carbonate de magnesium, le carbonate de cesium, le carbonate de strontium et les mélanges de ceux-ci, pour un pH de la solution aqueuse compris entre 7 et 10, de préférence entre 9,5 à 9,8 et à une température comprise entre 15 et 30 °C, de préférence environ 20 °C.

La présente invention a également pour objet un procédé de préparation du bromure de N-methyl naltrexone, comprenant au moins les étapes consistant à :
(i) faire réagir du methylsulfate de N-methyl naltrexone en solution aqueuse avec un agent alcalin choisi dans le groupe constitué par le carbonate de sodium, le carbonate de potassium, le carbonate de calcium, le carbonate de magnesium, le carbonate de cesium, le carbonate de strontium et les mélanges de ceux-ci, pour un pH du milieu réactionnel aqueux compris entre 7 et 10, de préférence entre 9,5 à 9,8 et à une température comprise entre 15 et 30 °C, de préférence environ 20°C, puis à
(ii) faire réagir le produit ainsi obtenu avec de l'acide bromhydrique, de préférence à 48 %, que l'on ajoute, pour un pH du milieu réactionnel aqueux compris entre 0,5 et 5, de préférence de l'ordre de 1, on maintient de préférence le contact sous agitation pendant encore une heure, pour ainsi obtenir le bromure de N-methyl naltrexone.

De préférence, l'agent alcalin est choisi dans le groupe constitué par le carbonate de sodium, le carbonate de potassium et les mélanges de ceux-ci.

Selon un mode de réalisation particulier, on peut ajouter du méthanol à la fin de l'étape (ii) décrite ci-dessus, on chauffe le milieu réactionnel à une température comprise entre 20 et 80 °C, par exemple entre 50 et 70°C, de préférence environ 60 °C, jusqu'à dissolution quasi-totale, puis on sépare par filtration le léger insoluble restant, pour refroidir ensuite le filtrat méthanol/eau, de préférence à environ 0°C, pour y faire cristalliser le bromure de N-methyl naltrexone souhaité.

Selon un autre mode de réalisation particulièrement préféré, le produit insoluble obtenu à la fin de l'étape (i) décrite ci-dessus est isolé après essorage, puis placé en suspension dans un mélange méthanol/eau, de préférence 4/1, constituant ainsi le milieu réactionnel aqueux pour l'étape (ii) dans laquelle la réaction avec l'acide bromhydrique, de préférence à 48 %, que l'on ajoute, pour un pH du milieu réactionnel aqueux compris entre 0,5 et 5, de préférence de l'ordre de 3, s'effectue à une température entre 20 et 80 °C, par exemple entre 50 et 70 °C, de préférence 60 °C, jusqu'à dissolution quasi-totale, on sépare ensuite par filtration le léger insoluble restant, pour refroidir ensuite le filtrat de préférence à environ 0°C, pour y faire cristalliser le bromure de N-methyl naltrexone souhaité.

La recristallisation dans un mélange méthanol/eau (du bromure de N-méthyl naltrexone) ou l'éventuel lavage du produit isolé (ion double N-méthyl naltrexone) par un solvant organique (méthanol par exemple) permet d'éliminer l'impureté lipophile O-benzyl N-méthyl naltrexone, bromure éventuellement encore présente.

Le procédé selon l'invention peut avantageusement comprendre une étape de purification du bromure de N-methyl naltrexone ainsi obtenu, par dissolution dans un mélange acétone/eau, de préférence 80/20, chauffage au reflux, de préférence pendant au moins environ 15 minutes, puis séparation par filtration à chaud, précipitation du bromure de N-methyl naltrexone par mise en contact du filtrat chaud avec de l'acétone chauffée, de préférence à environ 50 °C, refroidissement de milieu réactionnel à une température inférieure à 0°C, de préférence -2°C, le bromure de N-methyl naltrexone ainsi précipité étant récupéré par filtration, et séché.

Cette étape de purification du bromure de N-methyl naltrexone peut également être réalisée par dissolution dans un mélange méthanol/eau ou dans de l'eau seule, des rendements et des qualités similaires de la même entité chimique sont alors obtenus.

Dans le procédé décrit ci-dessous, le methylsulfate de N-methyl naltrexone peut être avantageusement obtenu en soumettant du methylsulfate de O-benzyl N-methyl naltrexone à une étape d'hydrogénation.

Cette étape d'hydrogénation peut avantageusement être réalisée comme décrit à l'exemple 1 ci-après, et d'une manière plus générale en soumettant du methylsulfate de O-benzyl N-methyl naltrexone, sous forme d'une solution aqueuse, à une hydrogénation sur charbon palladié à 5%, le milieu réactionnel étant porté à une température comprise entre 30 et 50 °C, de préférence 40 °C, sous une pression d'environ 2,5 bars d'hydrogène, pendant environ au moins 2 heures pour une O-débenzilation complète. Le milieu réactionnel est ensuite refroidi et le système catalytique éliminé par filtration.

Le produit obtenu peut avantageusement ne pas être isolé ce qui permet d'éviter tout contact avec le diméthylsulfate résiduaire (produit très toxique).

Dans le procédé selon l'invention, le methylsulfate de O-benzyle N-methyl naltrexone peut être avantageusement obtenu en faisant réagir du O-benzyl naltrexone avec du dimethylsulfate, dans de l'acétone, en présence d'hydrogénocarbonate de sodium, le milieu réactionnel étant porté au reflux pendant un temps suffisant, de préférence au moins environ 72 heures, pour une disparition acceptable du composé O-benzyl naltrexone, le suivi de la réaction pouvant être réalisé de manière connue par exemple par un suivi HPLC.

La présente invention a également pour objet le composé nouveau intermédiaire methylsulfate de O-benzyl N-methyl naltrexone, ainsi obtenu.

Les diastéréoisomères respectifs de configurations (R) et (S) vis-à-vis de l'atome d'azote du methylsulfate de O-benzyl N-methyl naltrexone, ainsi que leur mélanges, y compris les mélanges racémiques, font partie de l'invention.

En particulier, le groupe protecteur benzyle sur oxygène phénolique a tout particulièrement un double intérêt :
- clivage sans introduction et formation de produit ionique seul l'hydrogène est utilisé et le toluène formé est facilement éliminé ;
- l'hydrogénation permet de réduire la quantité de 7,8 didéhydro N-Méthyl Naltrexone (cétone conjuguée indésirable) dans le produit final après hydrogénation de la double liaison.

Par ailleurs, le procédé selon l'invention fournit une excellente diastéréosélectivité en amont et pour l'isolement sous forme ion double N-methyl naltrexone, et pour l'obtention du produit final souhaité à savoir la (R)- N-methyl naltrexone.

Dans le procédé selon l'invention, le O-benzyl naltrexone peut être avantageusement obtenu par réaction de chlorhydrate de naltrexone, ou de naltrexone base, avec du bromure de benzyle, dans de l'acétone, en présence de carbonate de potassium, le milieu réactionnel étant porté au reflux, à une température de préférence de l'ordre de 60°C, pendant environ 2 heures, puis on refroidit à température ambiante (environ 20°C) pour ensuite filtrer, éventuellement laver à l'acétone, et on évapore l'acétone du filtrat pour obtenir le composé souhaité sous forme d'une huile. De préférence, on reprend cette huile par exemple dans du dichlorométhane et on lave par exemple avec de la soude diluée (3%).

Cette extraction liquide en milieu basique permet d'éliminer totalement la naltrexone résiduaire non benzylée et d'éviter la formation du l'impureté 3-O- méthyl N-Méthyl Naltrexone dans l'étape d'alkylation / quaternisation.

Le produit peut avantageusement ne pas être isolé ce qui permet d'éviter la manipulation d'un milieu contenant du bromure de benzyle, produit lacrymogène et toxique.

Enfin, dans le procédé selon l'invention, le chlorhydrate de naltrexone ou la naltrexone base peut être avantageusement obtenu par réaction de chlorhydrate de noroxymorphone avec du bromométhylcyclopropane, dans du diméthylacétamide, en présence d'hydrogénocarbonate de sodium, le milieu réactionnel étant chauffé à une température comprise entre 60 et 75°C, de préférence entre 65 et 69°C, comme décrit par exemple à l'étape 1 du procédé de l'exemple 1.

La Figure 1 représente un diffratogramme (ou diagramme de poudre) théorique, obtenu à partir d'un monocristal de l'ion double, tel que décrit à l'exemple 1 (partie 1.5.2).

La Figure 2 représente un diffratogramme (ou diagramme de poudre) expérimental, obtenu à partir de l'ion double, tel que décrit à l'exemple 1 (partie 1.5.2).

Les exemples suivants sont destinés à illustrer la présente invention, de manière non limitative, et ne sauraient donc être interprêtés comme pouvant en restreindre la portée.

Sauf indication contraire, les données RMN ci-après sont obtenues avec la référence TMS (tétraméthylsilane).

### Exemple 1 : Préparation de bromure de N-méthyl Naltrexone

### 1.1 Préparation de Naltrexone base brute (Etape 1 ; N-Alkylation)

Dans un réacteur de 500 ml équipé d'un condenseur et d'une agitation mécanique, sont introduits successivement 100g (0,27 mol) de chlorhydrate de noroxymorphone, 80,8g (0,96 mol; 3,55 eq.),d'hydrogénocarbonate de sodium et 300ml de Diméthylacétamide. Le milieu réactionnel est chauffé entre 65°C et 69°C.

A la fin du dégagement gazeux observé, (environ 10 mn) 35 ml de brométhylcyclopropane (0,44 mol ; 1,6 eq) sont introduits en 30 mn en maintenant la température à 69°C.

La N- Alkylation est complète en environ 6 h et l'avancement de la réaction est contrôlé par analyse HPLC (tenue résiduaire en noroxymorphone inférieure ou égale à 0.5%). Le milieu réactionnel est refroidi à 50°C puis coulé sous agitation en 1 heure dans un mélange de 1000 ml d'eau et 100g de chlorure de sodium préalablement chauffé à 50°C.

Le pH est ajusté à 8,6-9 par addition de 8 ml de soude à 30%. Le produit obtenu est isolé par filtration à 15°C et séché en étuve sous vide à 50°C pendant 14 h.

On obtient finalement 86 g de Naltrexone brute (Rendement 88,6%) (conforme en HPLC par rapport au standard et conforme en structure RMN ¹H, ¹³C, masse).

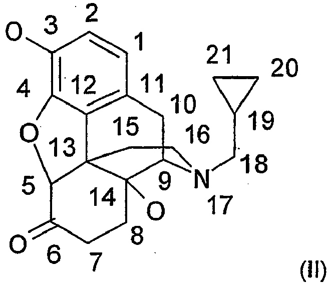

RMN ¹H : (ppm ±0,01 ppm) : 0,45 à 0,65 (2H, CH₂ (20/21), massif) ; 0,41 et 0,66 (2H, CH₂ (20/21), deux multiplets) ; 1,11 (1 H , CH (19), multiplet) ; 1,47 et 2,72 (2H, CH₂(15), multiplet et td) ; 1,50 et 2,05 (2H, CH₂ (8), deux multiplets); 2,10 et 3,03 (2H, CH₂ (7), deux multiplets); 2,48 et 3,03 (2H, CH₂ (16), deux multiplets); 2,97 et 3,32 (2H, CH2(18), deux multiplets) ; 4,02 (1 H, CH (9), doublet ; J = 6,0 H₂) ± 0,5 Hz) ; 5,04 (1H, CH (5), singulet) ; 6,71 (1H, CH(2), doublet ; J = 8 Hz ± 0,5 Hz) ; 7,11 (1H, COH (14), singulet) ; 9,05 (1H, NH, singulet) ; 9,05 (1H, COH (3), singulet).

RMN ¹³C (ppm ± 0,1ppm) : 2,6 et 5,0 (C20 et C21) ; 5,6 (C19) ; 22,8 (C10) ; 27,1 (C15) ; 30,6 (C8), 35 (C7) ; 46 (C16) ; 48,5 (C13) ; 56,6 (C18) ; 60,8 (C9) ; 69,7 (C14) ; 88,5 (C5) ; 118,0 (C2) ; 119,7 (C1) ; 120,4 (C11) ; 127,8 (C12) ; 140,1 (C4) ; 143,5 (C6).

Masse (ionisation chimique (MH)⁺ = 342,2

### 1.2 Préparation de O-Benzyle Naltrexone (Etape 2 ; O-Benzylation)

Dans un réacteur de 50 ml équipé d'un condenseur et d'une agitation mécanique sont ajoutés successivement 5,0g (0,014 mol)de Naltrexone, chlorhydrate (la base peut être utilisée) 5,0g (0,036 mol ; 2,58 eq) de carbonate de potassium et 25 ml d'acétone. 2,6 g (0,015 mol; 1,08 eq.) de bromure de benzyle sont ajoutés ensuite en 10 mn à 20°C sous agitation. Le milieu réactionnel est chauffé à reflux (60°C) pendant 2 h puis refroidi à 20°C et filtré. Le gâteau de filtration est lavé avec 2 fois 25 ml d'acétone.

L'acétone est évaporée sous vide et l'huile résiduelle est reprise dans 40 ml; de dichlorométhane puis lavée par 3 fois 25 ml de soude diluée (3%).

Cette extraction liquide en milieu basique permet d'éliminer totalement la naltrexone résiduaire non benzylée et d'éviter la formation du l'impureté 3-O-méthyl N-Méthyl Naltrexone dans l'étape 3 de quaternisation.

Après décantations et extractions la solution chlorométhylènique est concentrée jusqu'à absence de distillation puis engagée au stade suivant sans purification supplémentaire.

Le produit n'est pas isolé pour éviter la-manipulation d'un milieu contenant du bromure de benzyle qui est un produit lacrymogène et toxique.

Analyse structurale : on prélève un échantillon de l'huile obtenue pour isoler le produit O-benzyle Naltrexone sous forme de chlorhydrate (le chlorhydrate de O-benzyl Naltrexone est obtenu par mise en solution de la base sous forme d'huile dans le MTBE - ou Méthyl tert butyléther - et ajout d'acide chlorhydrique 35%).

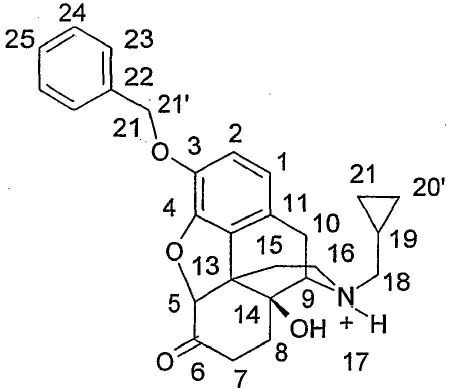

RMN ¹H (ppm ±0,01 ppm) : 1,2 (2H,CH₂ (20), multiplet J=6 Hz) ; 0,46 et 1,20 (2H,CH₂(20'), multiplet, J=5 Hz) ; 1,2 (1H, CH(19), multiplet, J=7,0 Hz); 3,2 (2H,CH₂,signaux larges) ; 1,67 et 3,2 (2H, C Hz (15), dd ; J=13,8 Hz, J=3,0 Hz, signaux larges) ; 1,64 et 2,51 (2H,C Hz(8), td, J=3,2 Hz, signaux larges) ; 2,33 et 3,25 (2H,CH₂(7), d, J=14,5 Hz, J=5,0, td J= 14,6 Hz, J=2,0 Hz) ; 2,51 et 3,45 (2H,CH₂ (16), signal large) ; 2,94 et 3,45 (2H,CH₂(18),dd ; J=12,5 Hz, J=7,2 Hz, signal large), 4,51(1H, CH (9), singulet large) ; 5,22 et 5,30 (2H, CH₂ (21) et CH2 (21') ; J=12,1 Hz) ; 5,00 (1H, CH (5) ; singulet large) ; 6,79 (1H, CH(2) et CH(1), système AB ; J= 8,3 Hz) ; 6,65 (1H, CH (1)et CH(2), système AB, J= 8,3 Hz) ; 6,65 (1 H, CH(23)CH(24) système benzylique) 6,65 (1 H, CH(25), système benzylique) ; 6,65 (1H,CH(24),CH(23), système benzylique).

RMN ¹³C (ppm± 0,1 ppm) : 3,8 (C20) ; (C20') ; 6,1(C19); 24,2 (C10); 27,5 (C15); 31,2 (C8); 35,4 (C7); 47,0 (C16); 49,2 (C13); ; 58,4 (C18); 61,2 (C9); 70,4 (C14); 72,1(C21 et 21'); 89,8 (C5); 118,9 (C2 et C1); 119,9 (C1 et C2); 121,6 (C22); 127,8 (C23 et C24); 128 (C25); 128,5 (C24 et C23); 137 (C3); 142,8 (C11 et C12); 145,9 (C12 et C11) ; 207,1 (C6)

Masse (ionisation MH⁺) = 432,5

### 1.3 Préparation de méthylsulfate de N-méthyl O-benzyle naltrexone (étape 3 : N-méthylation, quaternisation)

L'huile obtenue au stade précédent est dissoute dans 20 ml d'acétone puis coulée à 20°C sous agitation dans un réacteur sec de 50 ml contenant 1,3g (0,015 mol; 1,08 eq.) d'hydrogénocarbonate de sodium ; 6,7 g (0,053 mol; 3,53 eq.) de diméthylsulfate sont introduits ensuite en 10 min.

Le milieu réactionnel est chauffé sous agitation à reflux pendant 72 h minimum jusqu'à disparition totale de la O-benzyl Naltrexone (suivi HPLC).

Le milieu réactionnel est refroidi à 20°C puis filtré.

Le gâteau de filtration est lavé avec 2 fois (10 ml) d'acétone, puis mise en solution basique (NaHCO₃ ou NaOH). Ce filtrat est stocké à 20°C pour engagement au stade suivant sans isolement.

Le produit n'est pas isolé pour éviter la manipulation d'un produit contenant du diméthylsulfate. De même le gâteau de filtration (NaHCO₃ + reliquat diméthylsulfate) est dissout sur filtre sans isolement avec un milieu basique de façon à détruire le diméthylsulfate et former du méthylsulfate de sodium (non toxique).

Analyse structurale : on prélève une faible quantité du produit que l'on purifie en chromatographie préparative pour obtenir ainsi un échantillon analysé comme suit.

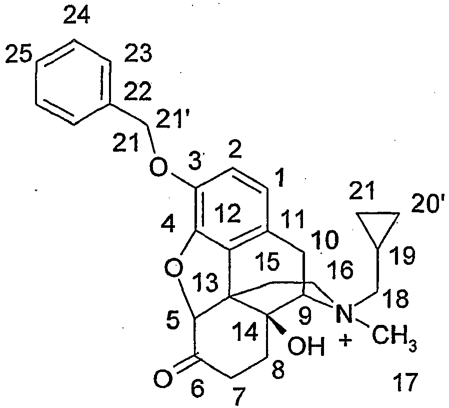

RMN ¹H (ppm ± 0,01 ppm) ; 0,41 et 0,88 (2H, CH₂ (20) ; multiplet , J= 5,0 Hz) ; 1,2 (1 H, CH (19), multiplet, J= 5,0 Hz) ; 0,55 et 1,06 (2H CH₂ (20') ; multiplet , J= 5,0 Hz) ; 1,75 et 3,0 (2H, CH₂ (15), d ; J= 12,5 Hz) ; 3,1 et 3,41 (2H, CH₂ (10), multiplet d, J= 5,5 Hz, J= 20,1 Hz) ; 1,63 et 2,43 (2H, CH₂ (8), td, doublet de multiplets, J= 13,7 Hz, J=3,2 Hz, J=11,5 Hz) ; 2,25 et 3,16 (2H, CH₂(7), dt, massif , J= 14,9 Hz ; J= 2,8 Hz) ; 3,66 (3H, CH₃ (17),s) ; 2,9 et 3,15 (2H, CH₂(16), multiplet, J= 3 Hz) ; 5,03 (1H,CH(9), d, J=4,1 Hz) 5,20 et 5,28 (2H, CH₂ (21) et (21', d, J= 12,0 Hz) ; 2,60 et 3,77 (2H, CH₂(18) ;dd,dd, J=13,5 Hz, J=9,4 Hz ; J=13,5 Hz, J=3,6 Hz) ; 5,05 (1H, CH(5), s) ; 6,82 (2H,CH(2) et CH(1), système AB, J= 8,3 Hz) ; 6,68 (2H,CH(1) et CH(2), système AB , J=8,3 Hz) ; 7,33 (2H, CH (23) et CH(24), système benzylique) 7,33 (1H, CH (25), système benzylique ; 7,33 (2H CH(23) et CH (24), système benzylique).

RMN ¹³C (ppm ± 0,1 ppm) : 3,6 (C20) ; 4,2 (C19) ; 7,1 (C20') ; 25 (C15) ; 27,9 (C10) ; 32,5 (C8) ; 35,3 (C7) ; 49,0 (C13) ; 53,8 (C17); 58 (C16); 71,4 (C9); 72 (C14); 7,21 (C21 et 21'); 73,2 (C18); 89,6 (C5); 119,0 (C2 et C1); 120,3 (C(1) et C(2)) ; 121,1 (C22) ; 127,8 (C23 et C24) 128,1 (C25) ; 128,5 (C24 et C23) ; 136,8 (C3); 143,3 (C11 et C12); 146,0 (C12 et C11) ; 206,8 (C6).

Masse (ionisation chimique M⁺) = 446

Par analyse HPLC, on constate l'existence des configurations respectives (R) et (S) vis à de l'atome d'azote, selon un rapport de configuration R/S de 96,6/3,4.

### 1.4 Préparation de méthylsulfate de N-méthyl naltrexone (étape 4: O-débenzylation)

La solution acétonique précédente est concentrée au tiers avant d'ajouter 100 ml d'eau et de poursuivre la distillation sous vide jusqu'à élimination de l'acétone

Après refroidissement à 20°C, la solution précédente est ajoutée à (0,3 g) de charbon palladié à 5%.

Le milieu réactionnel est alors porté à 40°C. Des séquences de purge (N₂ / H₂) sont faites avant établissement d'une pression de 2,5 bars d'hydrogène.

La O-Débenzylation est complète après environ 2 h suivi HPLC avec (teneur en N-Méthyl O-benzyl Naltrexone, méthylsulfate inférieur à 0.5%)-. Le milieu réactionnel est refroidi à 20°C et filtré pour éliminer le catalyseur.

La solution aqueuse de N-Méthylnaltrexone, méthylsulfate ainsi obtenue est engagée directement dans le stade suivant.

Le groupe protecteur benzyle sur oxygène phénolique à un double intérêt :
- clivage sans introduction et formation de produit ionique seul l'hydrogène est utilisé et le toluène formé est facilement éliminé.
- l'hydrogénation permet de réduire la quantité de 7,8 didéhydro N-Méthyl Naltrexone (cétone conjuguée donc structure alerte) dans le produit final après hydrogénation de la double liaison.

Le produit n'est pas isolé pour éviter le contact avec le diméthylsulfate résiduaire (produit très toxique).

### 1.5 Préparation de bromure de N-méthyl naltrexone (étape 5 : échange méthyl sulfate / bromure)

### 1.5.1 Ion double N-méthyl naltrexone (isolement de ce composé)

La solution aqueuse du stade 4 est concentrée sous vide jusqu'à obtenir un volume résiduel de 30 ml, on introduit ensuite 1g de Na₂CO₃ jusqu'à obtention d'un pH de l'ordre de 9,5 à 9,8 (pH naturel du carbonate de sodium dans l'eau).

Le milieu réactionnel est maintenu à 20°C sous agitation pendant 1 heure.

L'utilisation du carbonate de sodium dans cette étape permet en particulier de détruire le diméthylsulfate après 1 minute de contact.

L'insoluble formé est essoré et il apparaît ainsi qu'un ion double N-méthyl naltrexone peut exister dans ces conditions de pH particulières (avec l'utilisation de du carbonate de sodium Na₂ CO₃)

Analyse structurale : une partie de l'insoluble essoré obtenu ci-dessus est mis en suspension dans de l'eau à pH de l'ordre de 9,5 (ce qui permet de purifier l'ion double avant analyse par « dessalage ») puis est isolé par essorage et séchage.

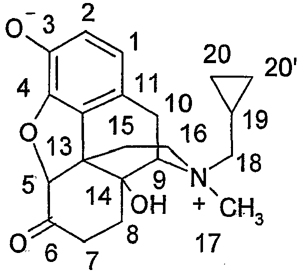

RMN ¹H (ppm ± 0,01 ppm) : 0,0 et 0,48 (2H, CH₂(C20)) ; multiplet, J=5,0 Hz, J=4,5 Hz) ; 0,88 (1H, CH (19), multiplet , J=4,0 Hz) ; 0,29 et 0,60 (2H,C Hz(20'), multiplet, J= 4,8 Hz) ; 1,49 et 2,51 (2H, CH₂ (15), doublet de multiplet , J=10,4 Hz) 2,79 et 3 ,29 (2H, CH₂(10), d, J= 19,9 Hz) ; 1,57 et 1,97 (2H, CH₂(8) ou (7) ; dd, doublet de multiplet, J=13,8 Hz, J= 3,9 Hz, J=15,2 Hz) ; 1,77 et 2,71 (2H, CH₂(7) ou (8), doublet de multiplet, dt, J= 13,9 Hz, J=14,9 Hz, J=5,4 Hz); 3,38 (3H,CH₃(17),s) ; 2,80 et 3,03 (2H,CH₂(16) ; dd ; J= 13,0 Hz, J= 3,5 Hz) ; 3,72 (1H, CH (9),d, J= 4,6 Hz) ; 2,47 et 3,60(2H,CH₂ (18) ; t ,dd, J=9,8 Hz, J=13,9 Hz, J=3,5 Hz) ; 4,54 (1H,CH(5),s), 6,35 (2H,CH(2) et CH(1), système AB, J= 8,2 Hz) ; 6,26 (2H, CH(1) et CH(2), système AB, J=8,1 Hz).

RMN ¹³(ppm ± 0,1 ppm) = 0,0 (C20) ; 1,3 (C19) ; 3,7 (C20') ; 22,2 (C15); 25,4 (C10); 30,2 (C8 ou C7); 30,3 (C7 ou C8); 47,0 (C13) ; 51,0 (C17) ; 55,5 (C16) ; 69,8 (C9) ; 70,3 (C18) ; 70,5 (C14) ; 111,9 (C5) ; 118,9 (C2 et C1) ; 119,6 (C1 et C2) ; 124,1 (C3) ; 143,8 (C11 et C12) : 147,8 (C12 et C11) ; 211,5 (C6).

Masse (ionisation chimique MH⁺) = 356

Analyse élémentaire :
- valeurs calculées théoriques (C 60,7% ; H 7,68% ; N 3,37% ; O 28,24%)
- valeurs expérimentales (C 61,64% ; H 7,6% ; N 3,19%).

Ces deux valeurs tiennent comptes d'une teneur en eau de 14,45% ce qui peut être interprété a priori comme un degré d'hydratation d'une forme trihydrate (3H₂O). Toutefois, on a encore effectué les analyses suivantes.

Analyse par diffraction de rayons X sur poudre (DRX) :

L'analyse est effectuée dans un diffractomètre D5005 de la société Brüker. Le domaine angulaire est compris entre 2,00 et 40,00°28 par pas de 0,02°20 et 2 secondes par pas. Le générateur est fixé à 50kV-40mA pour un tube en cuivre dont la longueur d'onde du faisceau incident est de 1,54056 Å.

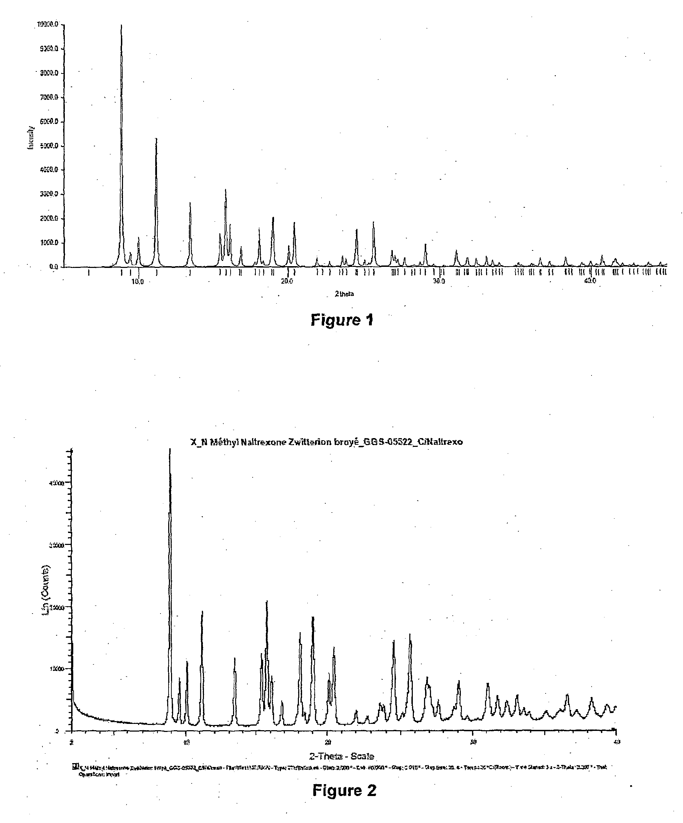

L'ion double purifié par « dessalage » comme décrit ci-dessus fourni un diffractogramme expérimental (voir Figure 2) qui s'avère être identique par comparaison à un diffractogramme théorique correspondant à une structure cristalline dihydrate (2H₂O). Ce diffractogramme théorique est obtenu par simulation (voir Figure 1 ; logiciel MERCURY® ) à partir des résultats d'une étude cristalline sur un monocristal du même ion double purifié par « dessalage ».

La différence de degré d'hydratation obtenue sur monocristal (2H₂O) et sur l'analyse élémentaire (3H₂O) s'explique par la présence de deux molécules d'eau de cristallisation dans la structure du réseau cristallin et d'une molécule d'eau provenant de l'eau d'insertion dans les micro-canaux des cristaux (eau d'imprégnation).

Par analyse HPLC, on constate l'existence des configurations respectives (R) et (S) vis à de l'atome d'azote, selon un rapport de configuration R/S de 98/2.

### 1.5.2 Bromure de N-méthyl naltrexone

L'insoluble précédent est placé en suspension dans 20 ml d'un mélange (4/1) MeOH / eau, de l'acide Bromhydrique est ajouté (q.s.p pH=3) puis le milieu réactionnel est porté à 60°C jusqu'à dissolution quasi totale.

Le léger insoluble (N-Méthyl Naltrexone non dissoute) est filtré puis le filtrat est refroidi à 0°C. Le bromure de N-méthyl naltrexone brut cristallise au refroidissement, il est ensuite essoré.

La recristallisation dans un mélange méthanol/eau (du bromure de N-méthyl naltrexone) ou l'éventuel lavage du produit isolé (« ion double ») par un solvant organique (méthanol par exemple) permet d'éliminer l'impureté lipophile O-benzyl N-méthyl naltrexone, bromure.

### 1.6 Préparation de bromure de N-méthyl naltrexone pure (étape 6: recristallisation dans acétone / eau)

Dans un réacteur de 50 ml équipé d'un condenseur, sont introduits successivement 5,6 g de N-Méthyl naltrexone, bromure brute (sèche), 7,5 ml d'eau et 22 ml d'acétone (soit 5 volumes du mélange 80:20 acétone/eau). Le milieu est porté à reflux pendant 15 min. Le trouble (N-méthyl Naltrexone, Bromure non dissoute) est filtré à chaud (60°C) et le filtrat chaud est versé sur 10 ml d'acétone à 50°C.

Le produit précipite en solution, la solution est refroidie à -2°C et le précipité filtré.

Le produit est séché sous vide à 20°C pendant 48 h.

On obtient finalement 4,3g de N-Méthyl Naltrexone, Bromure pur (rendement 76% par rapport à N-méthyl Naltrexone, bromure brut et 70% par rapport à Naltrexone, HCl de départ).

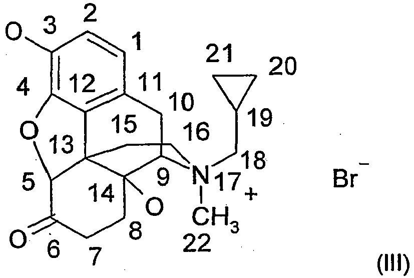

Caractéristiques physiques :
P fusion : (DSC) : 262°C
RMN 1H (ppm, ± 0,01) : identique à Naltrexone sauf 3,7 (3H, C(22) singulet) ; 13C (ppm +0,01) identique à Naltrexone sauf 58 (C (22)).
En tous points conformes avec les données de la littérature.

### Exemple 2 : Préparation de bromure de N-méthyl naltrexone (étape 5 : échange méthyl sulfate / bromure, variante sans isolement de l'intermédiaire

La solution aqueuse de l'étape 4 de l'exemple 1 est concentrée sous vide jusqu'à obtenir un volume résiduel de 30 ml, 1 g de Na₂CO₃ est introduit ensuite jusqu'à obtention d'un pH de l'ordre de 9,5 à 9,8 (pH naturel du carbonate de sodium dans l'eau).

Le milieu réactionnel est maintenu à 20°C sous agitation pendant 1 heure puis ajoute en 1 heure 2,1 ml d'acide bromhydrique 48% sont jusqu'à un pH de l'ordre de 1 et maintient le contact sous agitation pendant 1 heure supplémentaire.

L'insoluble du milieu réactionnel est essoré, et ce gâteau est lavé avec 10 ml d'acétone puis sécher en étuve sous vide (10 mm Hg) à 40°C pendant 12 h.

On obtient 9,35 g d'un mélange de bromure de N-méthyl naltrexone brut et de sels minéraux (NaBr et MeSO₄ Na ; titre en bromure de N-méthyl naltrexone brut 50%).

### Exemple 3: Préparation de bromure de N-méthyl naltrexone (étape 5: échange méthyl sulfate / bromure, variante sans isolement de l'intermédiaire, avec MeOH)

A l'étape 5 du procédé de l'exemple 1, on ajoute après traitement par HBr 40 m! de méthanol puis on porte l'ensemble à 60°C jusqu'à dissolution quasi totale. Le léger insoluble (N-Méthyl Naltrexone Bromure non dissoute) est filtré.

Le filtrat (mélange MeOH / H₂O) est refroidi à 0°C. Le bromure de MNTX brut cristallise au refroidissement puis est essoré.

L'avantage majeur de cette variante est la solubilisation de sels minéraux (NaBr, CH₃SO₄Na) dans le mélange méthanol/eau alors que NaBr est faiblement soluble dans le mélange éthanol/eau.

## Revendications

1. Ion double N-methyl naltrexone, de formule : 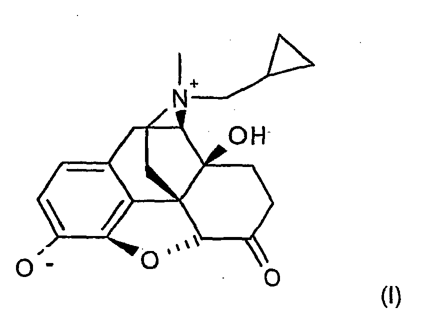 à l'état de forme anhydre ou d'hydrate.

2. Ion double N-methyl naltrexone selon la revendication 1, de configuration (R) vis-à-vis de l'atome d'azote.

3. Ion double N-methyl naltrexone selon la revendication 1, de configuration (S) vis-à-vis de l'atome d'azote.

4. Ion double N-methyl naltrexone selon l'une quelconque des revendications 1 à 3, à l'état d'hydrate choisi parmi l'état hémihydrate, dihydrate et trihydrate.

5. Ion double (R)-N-methyl naltrexone, dihydrate selon l'une des revendications 1, 2 ou 4.

6. Procédé de préparation du composé ion double N-methyl naltrexone, tel que défini à l'une quelconque des revendications précédentes, comprenant l'étape consistant à faire réagir du methylsulfate de N-methyl naltrexone en solution aqueuse avec un agent alcalin choisi dans le groupe constitué par le carbonate de sodium, le carbonate de potassium, le carbonate de calcium, le carbonate de magnesium, le carbonate de cesium, le carbonate de strontium et les mélanges de ceux-ci, pour un pH du milieu réactionnel aqueux compris entre 7 et 10.

7. Procédé de préparation du bromure de N-methyl naltrexone, comprenant au moins les étapes consistant à :
(i) faire réagir du methylsulfate de N-methyl naltrexone en solution aqueuse avec un agent alcalin choisi dans le groupe constitué par le carbonate de sodium, le carbonate de potassium, le carbonate de calcium, le carbonate de magnesium, le carbonate de cesium, le carbonate de strontium et les mélanges de ceux-ci, pour un pH du milieu réactionnel aqueux compris entre 7 et 10, puis à
(ii) faire réagir le produit ainsi obtenu avec de l'acide bromhydrique que l'on ajoute, pour un pH du milieu réactionnel aqueux compris entre 0,5 et 5 et ainsi obtenir le bromure de N-methyl naltrexone.

8. Procédé selon la revendication 6 ou 7, **caractérisée en ce que** l'agent alcalin est choisi dans le groupe constitué par le carbonate de sodium, le carbonate de potassium et les mélanges de ceux-ci.

9. Procédé selon la revendication 7 ou 8, dans lequel on ajoute du méthanol à la fin de l'étape (ii), on chauffe le milieu réactionnel à une température comprise entre 20 et 80 °C, puis on sépare par filtration l'insoluble restant, pour refroidir ensuite le filtrat dans lequel cristallise le bromure de N-methyl naltrexone souhaité.

10. Procédé selon l'une quelconque des revendications 7 à 9, dans lequel le produit insoluble obtenu à la fin de l'étape (i) est isolé après essorage, puis placé en suspension dans un mélange méthanol/eau, constituant ainsi le milieu réactionnel aqueux pour l'étape (ii) dans laquelle la réaction avec l'acide bromhydrique s'effectue à une température entre 20 et 80 °C, on sépare ensuite par filtration l'insoluble restant, pour refroidir ensuite le filtrat dans lequel cristallise le bromure de N-methyl naltrexone souhaité.

11. Procédé selon l'une quelconque des revendications 7 à 10, dans lequel on soumet le bromure de N-methyl naltrexone ainsi obtenu à une étape de purification par dissolution dans un mélange acétone/eau, chauffage au reflux puis séparation par filtration à chaud, précipitation du bromure de N-methyl naltrexone par mise en contact du filtrat chaud avec de l'acétone chauffée, refroidissement de milieu réactionnel à une température inférieur à 0°C, le bromure de N-methyl naltrexone ainsi précipité étant récupéré par filtration.

12. Procédé selon l'une quelconque des revendications 7 à 11, dans lequel le methylsulfate de N-methyl naltrexone est obtenu en soumettant du methylsulfate de O-benzyl N-methyl naltrexone à une étape d'hydrogénation.

13. Procédé selon la revendication 12, dans lequel le methylsulfate de O-benzyle N-methyl naltrexone est obtenu en faisant réagir du O-benzyl naltrexone avec du dimethylsulfate, dans de l'acétone, en présence d'hydrogénocarbonate de sodium, le milieu réactionnel étant porté au reflux pendant un temps suffisant pour une disparition acceptable du composé O-benzyl naltrexone.

14. Methylsulfate de O-benzyl N-methyl naltrexone.

15. Methylsulfate de O-benzyl N-methyl naltrexone selon la revendication 14, de configuration (R) vis-à-vis de l'atome d'azote.

16. Methylsulfate de 0-benzyl N-methyl naltrexone selon la revendication 14, de configuration (S) vis-à-vis de l'atome d'azote.

17. Procédé selon la revendication 13, dans lequel le O-benzyl naltrexone est obtenu par réaction de chlorhydrate de naltrexone, ou de naltrexone base, avec du bromure de benzyle, dans de l'acétone, en présence de carbonate de potassium, le milieu réactionnel étant porté au reflux, puis on refroidit pour ensuite filtrer, et on évapore l'acétone du filtrat pour obtenir le composé souhaité sous forme d'une huile.

18. Procédé selon la revendication 17, dans lequel le chlorhydrate de naltrexone ou la naltrexone base est obtenu par réaction de chlorhydrate de noroxymorphone avec du brométhyl cyclopropane, dans du diméthylacétamide, en presence d'hydrogénocarbonate de sodium, le milieu réactionnel étant chauffé à une température comprise entre 60 et 75°C.

## Patentansprüche

1. N-Methylnaltrexon-Zwitterion der Formel: 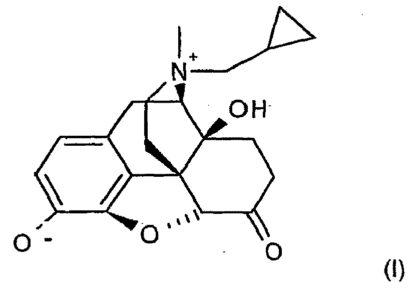 in wasserfreier Form oder Hydratform.

2. N-Methylnaltrexon-Zwitterion nach Anspruch 1 mit (R)-Konfiguration in Bezug auf das Stickstoffatom.

3. N-Methylnaltrexon-Zwitterion nach Anspruch 1 mit (S)-Konfiguration in Bezug auf das Stickstoffatom.

4. N-Methylnaltrexon-Zwitterion nach einem der Ansprüche 1 bis 3 in Hydratform, ausgewählt aus der Hemihydrat-, Dihydrat- und Trihydratform.

5. (R)-N-Methylnaltrexon-2witterion-dihydrat nach einem der Ansprüche 1, 2 oder 4.

6. Verfahren zur Herstellung der N-Methylnaltrexon-Zwitterion-Verbindung gemäß einem der vorhergehenden Ansprüche, bei dem man N-Methylnaltrexon-methylsulfat in wässriger Lösung mit einem aus der Gruppe bestehend aus Natriumcarbonat, Kaliumcarbonat, Calciumcarbonat, Magnesiumcarbonat, Caesiumcarbonat, Strontiumcarbonat und Mischungen davon ausgewählten alkalischen Mittel für einen pH-Wert des wässrigen Reaktionsmediums zwischen 7 und 10 umsetzt.

7. Verfahren zur Herstellung von N-Methylnaltrexonbromid, bei dem man mindestens:
(i) N-Methylnaltrexon-methylsulfat in wässriger Lösung mit einem aus der Gruppe bestehend aus Natriumcarbonat, Kaliumcarbonat, Calciumcarbonat, Magnesiumcarbonat, Caesiumcarbonat, Strontiumcarbonat und Mischungen davon ausgewählten alkalischen Mittel für einen pH-Wert des wässrigen Reaktionsmediums zwischen 7 und 10 umsetzt und dann
(ii) das so erhaltene Produkt mit Bromwasserstoffsäure, die für einen pH-Wert des wässrigen Reaktionsmediums zwischen 0,5 und 5 zugesetzt wird, umsetzt und so das N-Methylnaltrexon-bromid erhält.

8. Verfahren nach Anspruch 6 oder 7, **dadurch gekennzeichnet, dass** das alkalische Mittel aus der Gruppe bestehend aus Natriumcarbonat, Kaliumcarbonat und Mischungen davon ausgewählt wird.

9. Verfahren nach Anspruch 7 oder 8, bei dem man am Ende von Schritt (ii) Methanol zugibt, das Reaktionsmedium auf eine Temperatur zwischen 20 und 80°C erhitzt, dann die verbleibende unlösliche Substanz abfiltriert und danach das Filtrat abkühlt, aus dem das gewünschte N-Methylnaltrexonbromid kristallisiert.

10. Verfahren nach einem der Ansprüche 7 bis 9, bei dem man das am Ende von Schritt (i) erhaltene unlösliche Produkt nach Absaugen isoliert und dann in einer Methanol/Wasser-Mischung suspendiert, welche somit das wässrige Reaktionsmedium für Schritt (ii) bildet, in dem die Umsetzung mit der Bromwasserstoffsäure bei einer Temperatur zwischen 20 und 80°C stattfindet, und danach die verbleibende unlösliche Substanz abfiltriert und danach das Filtrat abkühlt, aus dem das gewünschte N-Methylnaltrexon-bromid kristallisiert.

11. Verfahren nach einem der Ansprüche 7 bis 10, bei dem man das so erhaltene N-Methylnaltrexon-bromid einem Reinigungsschritt durch Lösen in einer Aceton/Wasser-Mischung, Erhitzen zum Rückfluss und dann Heißfiltration, Ausfällen des N-Methylnaltrexon-bromids durch Inberührungbringen des heißen Filtrats mit erhitztem Aceton und Abkühlen des Reaktionsmediums auf eine Temperatur von weniger als 0°C unterwirft, wobei das so ausgefällte N-Methylnaltrexon-bromid durch Filtration gewonnen wird.

12. Verfahren nach einem der Ansprüche 7 bis 11, bei dem N-Methylnaltrexon-methylsulfat erhalten wird, indem man O-Benzyl-N-methylnaltrexon-methylsulfat einem Hydrierungsschritt unterwirft.

13. Verfahren nach Anspruch 12, bei dem das O-Benzyl-N-methylnaltrexon-methylsulfat durch Umsetzung von O-Benzylnaltrexon mit Dimethylsulfat in Aceton in Gegenwart von Natriumhydrogencarbonat erhalten wird, wobei das Reaktionsmedium über einen für ein annehmbares Verschwinden der O-Benzylnaltrexon-Verbindung ausreichenden Zeitraum zum Rückfluss gebracht wird.

14. O-Benzyl-N-methylnaltrexon-methylsulfat.

15. O-Benzyl-N-methylnaltrexon-methylsulfat nach Anspruch 14 mit (R)-Konfiguration in Bezug auf das Stickstoffatom.

16. O-Benzyl-N-methylnaltrexon-methylsulfat nach Anspruch 14 mit (S)-Konfiguration in Bezug auf das Stickstoffatom.

17. Verfahren nach Anspruch 13, bei dem das O-Benzylnaltrexon durch Umsetzung von Naltrexon-hydrochlorid oder Naltrexon-Base mit Benzylbromid in Aceton in Gegenwart von Kaliumcarbonat erhalten wird, wobei das Reaktionsmedium zum Rückfluss gebracht, dann abgekühlt und danach filtriert wird und das Aceton von dem Filtrat abgedampft wird, wobei man die gewünschte Verbindung in Form eines Öls erhält.

18. Verfahren nach Anspruch 17, bei dem das Naltrexonhydrochlorid bzw. die Naltrexon-Base durch Umsetzung von Noroxymorphon-hydrochlorid mit Brommethylcyclopropan in Dimethylacetamid in Gegenwart von Natriumhydrogencarbonat erhalten wird, wobei das Reaktionsmedium auf eine Temperatur zwischen 60 und 75°C erhitzt wird.

## Claims

1. N-Methylnaltrexone double ion, of formula: 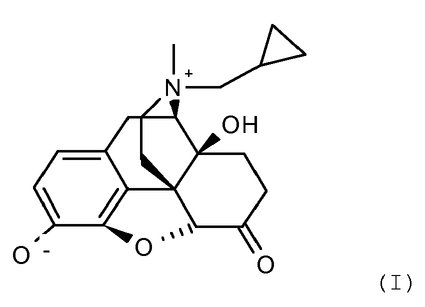 in anhydrous or hydrate form.

2. N-methylnaltrexone double ion according to Claim 1, of (R) configuration relative to the nitrogen atom.

3. N-methylnaltrexone double ion according to Claim 1, of (S) configuration relative to the nitrogen atom.

4. N-methylnaltrexone double ion according to any one of Claims 1 to 3, in the form of a hydrate chosen from the hemihydrate, dihydrate and trihydrate forms.

5. (R)-N-methylnaltrexone double ion, dihydrate according to one of claims 1, 2 or 4.

6. Process for preparing the N-methylnaltrexone double ion compound, as defined in any one of the preceding claims, comprising the step that consists in reacting N-methylnaltrexone methyl sulfate in aqueous solution with an alkaline agent chosen from the group constituted by sodium carbonate, potassium carbonate, calcium carbonate, magnesium carbonate, caesium carbonate, strontium carbonate and mixtures thereof, for a pH of the aqueous reaction medium of between 7 and 10.

7. Process for preparing N-methylnaltrexone bromide, comprising at least the steps consisting in:
(i) reacting N-methylnaltrexone methyl sulfate in aqueous solution with an alkaline agent chosen from the group constituted by sodium carbonate, potassium carbonate, calcium carbonate, magnesium carbonate, caesium carbonate, strontium carbonate and mixtures thereof, for a pH of the aqueous reaction medium of between 7 and 10, and then in
(ii) reacting the product thus obtained with hydrobromic acid, which is added for a pH of the aqueous reaction medium of between 0.5 and 5, in order thus to obtain the N-methylnaltrexone bromide.

8. Process according to Claim 6 or 7, **characterized in that** the alkaline agent is chosen from the group constituted by sodium carbonate and potassium carbonate, and mixtures thereof.

9. Process according to Claim 7 or 8, in which methanol is added at the end of step (ii), the reaction medium is heated to a temperature of between 20 and 80°C, and the remaining insoluble matter is then separated out by filtration, in order subsequently to cool the filtrate, from which the desired N-methylnaltrexone bromide crystallizes.

10. Process according to any one of Claims 7 to 9, in which the insoluble product obtained at the end of step (i) is isolated after filtration by suction, and is then suspended in a methanol/water mixture, thus constituting the aqueous reaction medium for step (ii) in which is performed the reaction with hydrobromic acid at a temperature of between 20 and 80°C, and the remaining insoluble matter is then separated out by filtration, in order subsequently to cool the filtrate, from which the desired N-methylnaltrexone bromide crystallizes.

11. Process according to any one of Claims 7 to 10, in which the N-methylnaltrexone bromide thus obtained is subjected to a purification step by dissolution in an acetone/water mixture, heating to reflux and then separation by hot filtration, precipitation of the N-methylnaltrexone bromide by placing the hot filtrate in contact with warm acetone, cooling of the reaction medium to a temperature below 0°C, the N-methylnaltrexone bromide thus precipitated being recovered by filtration.

12. Process according to any one of Claims 7 to 11, in which the N-methylnaltrexone methyl sulfate is obtained by subjecting O-benzyl-N-methylnaltrexone methyl sulfate to a hydrogenation step.

13. Process according to Claim 12, in which the O-benzyl-N-methylnaltrexone methyl sulfate is obtained by reacting O-benzylnaltrexone with dimethyl sulfate, in acetone, in the presence of sodium hydrogen carbonate, the reaction medium being refluxed for a sufficient time for acceptable disappearance of the O-benzylnaltrexone compound.

14. O-Benzyl-N-methylnaltrexone methyl sulfate.

15. O-Benzyl-N-methylnaltrexone methyl sulfate according to Claim 14, of (R) configuration relative to the nitrogen atom.

16. O-Benzyl-N-methylnaltrexone methyl sulfate according to Claim 14, of (S) configuration relative to the nitrogen atom.

17. Process according to Claim 13, in which the O-benzylnaltrexone is obtained by reacting naltrexone hydrochloride, or base naltrexone, with benzyl bromide, in acetone, in the presence of potassium carbonate, the reaction medium being maintained at reflux, and then cooled in order subsequently to filter, and the acetone is evaporated from the filtrate to obtain the desired compound in the form of an oil.

18. Process according to Claim 17, in which the naltrexone hydrochloride or the base naltrexone is obtained by reacting noroxymorphone hydrochloride with bromomethylcyclopropane, in dimethylacetamide, in the presence of sodium hydrogen carbonate, the reaction medium being heated to a temperature of between 60 and 75°C.
